# EUROPÄISCHE PATENTANMELDUNG

(11) **EP 3 178 476 A1**
(43) Veröffentlichungstag der Anmeldung: **14.06.2017**
(21) Anmeldenummer: 16206219.4
(22) Anmeldetag: 10.06.2010
(51) Int. Cl.: A61K 31/196, A61K 31/405, A61K 31/567, A61K 45/06, A61P 5/34

(54) **PHARMAZEUTISCHE ZUSAMMENSETZUNG UND BEHANDLUNGSVERFAHREN ZUR NOTFALLEMPFÄNGNISVERHÜTUNG**

(30) Priorität: 23.06.2009 DE 102009030607
(62) Teilanmeldung aus: 10722968.4
(71) Anmelder: Bayer Intellectual Property GmbH, 40789 Monheim am Rhein (DE)
(72) Erfinder: Lindenthal, Bernhard, 13507 Berlin (DE); Schuermann, Rolf, 14513 Teltow (DE); General, Sascha, 13158 Berlin (DE)
(74) Vertreter: BIP Patents

(57) **Zusammenfassung**

Die Erfindung betrifft, pharmazeutische Zusammensetzungen für Notfallverhütung, die Verwendung von Levonorgestrel in Kombination mit Cox Inhibitoren für die Herstellung von pharmazeutischen Zusammensetzungen für den genannten Zweck, sowie ein Verfahren zur Herstellung dieser pharmazeutischen Zusammensetzungen.

## Beschreibung

Die Erfindung betrifft, pharmazeutische Zusammensetzungen für Notfallverhütung (auch bekannt unter den Bezeichnungen "Day After Pill", oder "Emergency contraception"), die Verwendung von Levonorgestrel in Kombination mit Cox Inhibitoren für die Herstellung von pharmazeutischen Zusammensetzungen für den genannten Zweck, sowie ein Verfahren zur Herstellung dieser pharmazeutischen Zusammensetzungen.

Verschiedene Vorrichtungen und pharmazeutische Zusammensetzungen, wie zum Beispiel das Kondom, Pessar, Intrauterinpessar sowie die verschiedenen oralen Mono- oder Multiphasen-Verhütungsmittel, stehen für die Verhinderung von ungewünschter Schwangerschaft zur Verfügung. Gleichwohl wird trotz der verschiedensten Verhütungsmethoden, der Koitus in vielen Fällen ungeschützt vollzogen, auch wenn eine Schwangerschaft nicht gewollt ist.

Eine solche Situation besteht zum Beispiel bei Vergewaltigungsopfern oder wenn die Vorrichtung für die Empfängnisverhütung, z.B. das Kondom, beschädigt ist. Sofern in diesen Fällen die Ovulation oder - falls die Ovulation bereits stattgefunden hat - die Fertilisierung verhindert wird, kann eine Schwangerschaft vermieden werden. Zu diesem Zweck, muss die Notfallverhütung zeitnah nach dem Koitus, spätestens innerhalb von 72 Stunden, durchgeführt werden.

Für diesen Fall der Notfallverhütung finden neben der Intrauterinen Kontrazeption mit kupferhaltigen Intrauterinpessaren (z.B. Nova T^{®}) vor allem die sogenannten Emergency Contraception Pills (ECPs) Anwendung, wobei hier zwei Typen zu unterscheiden sind: (a) EPC's, die sowohl Östrogene als auch Gestagene enthalten und (b) die neueren "Progestin-only" Pillen, die ausschließlich Gestagen als wirksamen Bestandteil enthalten.

Die neueren "Progestin only" ECPs haben inzwischen die älteren kombinierten ECPs weitgehend ersetzt, weil sie effizienter sind und weniger Nebenwirkungen verursachen. Gleichwohl liegt die empfängnisverhütende Wirksamkeit auch dieser Präparate deutlich unter der Effizienz, wie sie durch regelmäßige Einnahme eines oralen Kontrazeptivums erreicht wird. So berichtet Trussel et al. in seinem Artikel "Understanding Contraceptive Failure" [Best Practice & Research Clinical Obstetrics and Gynaecology 23 (2009) 199-209], dass in unterschiedlichen Studien die Effektivität von LNG basierten EPCs nur zwischen 59 und 94% lag.

Der Wirkmechanismus dieser Östrogen und Gestagen enthaltenden Präparate als auch der Präparate, die ausschließlich Gestagen enthalten, wurde in einer Vielzahl von Studien untersucht. Diese Studien belegen, dass der Wirkmechanismus in einer Hemmung bzw. Verzögerung der Ovulation besteht^{1,2,3,4}.

Diese Verzögerung der Ovulation erklärt die Wirksamkeit der ECPs, sofern sie während der ersten Hälfte des Zyklus, d.h. vor dem Eisprung verabreicht werden.

Ob EPC's auch nach der Ovulation einen Effekt auf die Vermeidung einer Schwangerschaft ausüben, wurde ebenfalls in Studien untersucht. Eine umfassende Übersicht hierzu geben J. Trussell und E. G. Raymond in ihrem im März 2009 veröffentlichten Übersichtsartikel "Emergency Contraception: A Last Chance to Prevent Unintended Pregnancy". Die Autoren kommen aufgrund der verschiedenen, zum Teil widersprechenden (Studien-) Ergebnisse jedoch zu der Schlussfolgerung, dass die Frage, ob ECP's eine Schwangerschaft auch nach der Fertilisierung verhindern können, wohl nie eindeutig zu klären sein wird.

So zeigen einige Studien histologische oder biochemische Veränderungen in der Gebärmutterschleimhaut nach Behandlung mit der ECP. Diese lassen den Schluss zu, dass ECPs auch die Einnistung des befruchteten Eis in das Endometrium beinträchtigen können^{1,5,6,7}.

Jüngere Studien widersprechen jedoch dieser Annahme, dass die Einnahme ECP Auswirkungen auf das Endometrium hat^{1,8,9}

Als weitere Effekte werden eine Störung der Corpus Luteum Funktion, Verdickung des Zervixschleims mit Einfluss auf den Zugang der Spermien, eine Veränderung des tubaren Transportes der Spermien bzw. des Eis und die direkte Hemmung der Fertilisierung diskutiert^{13,10,11,12}.

Gleichwohl deuten statistische Daten über die Wirksamkeit von ECPs darauf hin, dass verschiedene Faktoren zur Wirksamkeit beitragen und deren Wirkung nicht alleine auf eine Verzögerung oder Verhinderung der Ovulation zurückzuführen ist¹⁴.

Einige Studien haben gezeigt, dass die frühzeitige Behandlung mit ECPs, die nur das Gestagen (Levonorgestrel) enthalten, einen Einfluss sowohl auf den ovulatorischen Prozess als auch auf die luteale Funktion haben ^{15,18,19,20,58}. Zwei andere Studien zeigen hingegen keine Auswirkung auf das Endometrium^{16,17}.

Eine andere Studie, bei der das Gestagen (Levonorgestrel) vor dem LH-Anstieg verabreicht wurde, zeigt wiederum einen Einfluss auf das sekretorische Muster von Glycodelin im Serum und das Endometrium²¹. Allerdings konnte dieses Ergebnis in einer späteren Studie, die ausdrücklich die Beurteilung der endometrialen Glycodelin Expression zum Ziel hatte²², nicht bestätigt werden.

In einer vor mehr als 30 Jahren durchgeführten Studie mit Levonorgestrel wurde ein Einfluss auf die Wanderung der Spermien und die Funktion des Genitaltraktes festgestellt²³. Eine aktuellere Studie hierzu ergab jedoch, dass 1,5 mg Levonorgestrel keine Auswirkungen auf die Zervixschleimhaut oder auf das Eindringen der Spermien in die Gebärmutterhöhle hat²².

Die reduzierte Wirksamkeit der EPC's bei verzögerter Einnahme nach dem ungeschützen Verkehr legt nahe, dass EPC's keinen Einfluss auf die Einnistung der Eizelle haben, denn ansonsten dürfte die Wirksamkeit des Präparates nicht vom Einnahmezeitpunkt abhängig sein, zumindest solange wie die Einnahme der ECP vor der Einnistung erfolgt²⁴.

Studien an Ratten und Cebus (Kapuzineraffen) mit Levonorgestrel in einer ovulationshemmenden Dosis zeigen, dass die Fertilität nach Fertilisierung nicht beeinträchtigt beeinträchtigt wird^{12,25,26}. Ob diese Beobachtung auf den Menschen übertragbar ist, bleibt jedoch offen.

Obgleich der Wirkmechanismus von ECPs nicht vollständig geklärt ist, ist bewiesen, dass ECPs keine abortive Wirkung, auch im Sinne der Definitionen der medizinischen Behörden, wie z.B. der Food and Drug Administration / National Institutes of Health, haben²⁷.

Neben den Studien zur Untersuchung des Wirkmechanismus, wurde die Eignung verschiedener Behandlungsregime, Gestagene und Dosen zur postkoitalen Verhütung ausgiebig untersucht. Postkoitale Verhütung bedeutet, dass Frauen, die nicht schwanger werden wollen, nach dem ungeschützten Koitus, entsprechende pharmazeutische Präparate anwenden, die eine Ovulation verhindern sollen.

Erste Arbeiten hierzu wurden bereits in den 70er Jahren durchgeführt. So wurden verschiedene Gestagene, unter anderem Levonorgestrel, als postkoitale Routineverhütungsmittel in umfassenden Studien getestet (The Journal of Reproductive Medicine, 13(2), (1974); Contraception, 7(5), 367-379, (1973); Reproduction, 2(1), 61-62, (1975); International Journal of Fertility, 20, 156-1 60, (1975)]. Die Einzeltagesdosen lagen zwischen 150 µg und 1.500 µg. Die Ergebnisse der Studien zeigten, dass die postkoitale Verhütungseffizienz von Levonorgestrel - wenn es allein verwendet wird - selbst als 1 mg Dosis gering war.

A. A. Yuzpe und Mitarbeiter [The Journal of Reproductive Medicine 13(2), (1974)] berichteten Ergebnisse solcher Studien, worin eine pharmazeutische Zusammensetzung, die 100 µg Ethinylestradiol und 1,0 mg Norgestrel enthält, als postkoitales Verhütungsmittel in einer Einzeldosis verwendet wurde. Die Zusammensetzung wurde innerhalb von fünf Tagen nach dem ungeschützten Koitus verabreicht. Später wurde dieses Verfahren abgeändert. Auf der einen Seite wurde die Zeitdauer der möglichen Verwendung der Zusammensetzung von 5 Tagen auf 72 Stunden reduziert, auf der anderen Seite wurde die Dosis auf dem Weg verdoppelt, dass die Verabreichung 12 Stunde nach der ersten wiederholt wurde [Fertility and Sterility, 28, 932-936, (1977); ibid. 37, 508-51 3 (1982); International Journal of Gynaecology and Obstetrics, 15, 133-1 36, (1977)]. Diese Modifikation verbesserte den Erfolg des Verfahrens.

Nach den Studien von A. A. Yuzpe und Mitarbeitern wurden verschiedene andere Versuche durchgeführt, um die Wirksamkeit dieser Kombination nachzuweisen. In diesen Studien war die Gesamtdosis an Ethinyl-estradiol 0,2 mg, kombiniert mit 2,0 mg Norgestrel oder 1,0 mg Levonorgestrel. Die Ergebnisse der Studien zeigten, dass, obwohl die obige Verabreichung (Yuzpe-Regime) weniger Nebenwirkungen verursachte als Östrogene, die früher in höheren Dosen verwendet wurden, das relative Auftreten von Übelkeit und Erbrechen immer noch sehr hoch war (jeweils 50 und 20%). Diese Nebenwirkungen beruhen auf der Östrogenwirkung und führen zu einer verringerte Akzeptanz der Methode. Darüber hinaus sinkt die Wirksamkeit der Behandlung, wenn Erbrechen auftritt.

Die Verwendung von Levonorgestrel für Notfallverhütung wurde in den 90-ziger Jahren entdeckt. Die Ergebnisse der Studien wurden in zwei gut dokumentierten Publikationen berichtet [Lancet, 352, 428-433, (1998)] und Human Reproduction, 8(3), 389-392, (1993)]. Die Wirksamkeit von Tabletten, die nur 0,75 mg Levonorgestrel enthalten und von Kombinationstabletten der Yuzpe-Methode, die 0,1 mg Ethinylestradiol und 1,0 mg Levonorgestrel enthalten, wurde bei der 12 Stunden getrennten Verabreichung der Dosen innerhalb von 48 sowie innerhalb von 72 Stunden nach dem ungeschützten Koitus untersucht. Die Ergebnisse zeigten, dass der Schutz mit zwei Tabletten, die 0,75 mg Levonorgestrel enthalten, besser war als der mit dem Yuzpe-Regime, und das darüber hinaus bei den Frauen, die nur Levonorgestrel erhielten, weniger Nebenwirkungen auftraten, was auf das Fehlen von Ethinylestradiol zurückzuführen sein könnte.

Die Ergebnisse der klinischen Studien zeigten auch, dass die Wirkung besser war, je früher die Behandlung nach dem Koitus begonnen wurde. Die Erfahrung zeigt allerdings, dass Frauen die Einnahme der ersten Tablette verzögerten, um zu vermeiden, dass die Einnahme der zweiten Dosis nach 12 Stunden in eine ungünstige Zeit fällt (zum Beispiel in die Schlafphase). Gemäß der Studien ist eine genaue Einhaltung des 12 Stunden Intervalls zwischen den zwei Dosen aber essentiell, um die gewünschte Wirkung nicht zu verringern. Gemäß statistischer Daten nahm der Großteil der Frauen die zweite Dosis innerhalb von 12 bis 16 Stunden nach der ersten Einnahme ein [Lancet, 352, 428-433, (1998)].

Aufgrund des genannten Nachteils (der verzögerten Einnahme der 2-ten Tablette) beschreibt das Europäische Patent 1448207 ein Präparat, das nur aus einer Tablette besteht, die jedoch die doppelte Menge an Levonorgestrel, also 1,5 mg Wirkstoff enthält. Diese Tablette muss innerhalb von 72 Stunden nach ungeschütztem Koitus verabreicht werden. Wie in einer klinischen Studie gezeigt werden konnte, ist die empfängnisverhütende Wirkung vergleichbar oder sogar geringfügig besser, als bei der Aufteilung der Dosis auf 2 Tabletten zu je 0,75 mg [E. Jonannson et al, Human Reproduction, Vol. 17, No. 6, 1472-1476 (2002)], Johannsen nimmt jedoch eine schlechtere Verträglichkeit der hoch dosierten 1,5 mg Formulierung an (Übelkeit, Unwohlsein, Erbrechen sowie eine Störung des Menstruationszyklus).

Andere Forschungsgruppen haben den Einfluss von COX-Inhibitoren auf die Ovulation untersucht. So untersuchten Pall et al. den Einfluss von Rofecoxib auf die Ovulation [Pall et al.; Human Reproduction Vol 16, Nr. 7, pp 1323-1328 (2001)]. In dieser Studie wurden 25 mg des Wirkstoffs an 9 aufeinander folgenden Tagen eingesetzt. Eine Verzögerung des Eisprungs von mehr als 48 Stunden wird in 4 von 6 Patientinnen erreicht.

Die Studie von M.S. Bata et al. liegt in Einklang mit den Ergebnisse von Pall, untersucht jedoch den Einfluss von Meloxicam auf die Ovulation [Bata et al., J Clin Pharmacol (2006) 46:925-932]. Diese Studie zeigt, dass eine Dosis von 30 mg pro Patient und Tag (an 5 aufeinander folgenden Tagen) eine Verzögerung des Eisprungs von 5 Tagen erreicht werden kann.

Cox-Inhibitoren sind grundsätzlich als relativ gut verträglich bekannt, jedoch weist die EMEA [EMEA/62838/2005; EMEA/62757/2005] auf ein für diese Substanzklasse erhöhtes Risiko für unerwünschte kardiovaskuläre Ereignisse hin und empfiehlt daher die niedrigste effektive Dosis anzuwenden. Die aktuelle Studiendatenlage zeigt dabei einen Trend, dass mit steigender Selektivität für COX-2 (Cyclooxygenase Typ 2) das Risiko für renale und kardiovaskuläre Ereignisse steigt. Meloxicam ist daher im Vergleich zu anderen Cox-Inhibitoren wie z.B. Piroxicam als eher kritisch anzusehen [Clin Pharmacol Ther. 2009 Feb;85(2):190-7; Pharmacotherapy 2006;26(7):919-938]. Eine steigende COX-2 Selektivität geht jedoch einher mit geringeren gastrointestinalen Nebenwirkungen, wobei unerwünschte kardiovaskuläre Ereignisse bisher nur bei chronischer Anwendung gefunden wurden [Am. J. Med. 2004;117:100 -106].

Massai et al. untersuchte in einer Pilotstudie die Verwendung von Meloxicam in Kombination mit Levonorgestrel (LNG) auf den Ovulationspunkt im Zusammenhang mit der Emergency Contraception [Human Reproduction Vol. 22, Nr. 2, pp 434-439 (2007)]. In dieser Studie wurden 2 Tabletten mit jeweils 0,75 mg LNG (u.a. bekannt unter dem Handelsnamen Postinor-2) eingesetzt. Meloxicam wurde in einer Dosis von 15 mg angewendet. In der Patientengruppe, die die Kombination aus Meloxicam und LNG erhalten hatten, gab es einen Trend zu einer verringerten Häufigkeit des Eisprungs im Vergleich zur der Gruppe, die ausschließlich mit LNG behandelt wurde. Dieser Effekt war zunehmend stärker, je später die Tabletten relativ zum Eisprung eingenommen wurden.

Die Aufgabe dieser Erfindung ist es daher eine pharmazeutische Zusammensetzung für Notfallverhütung bereitzustellen, die bei Einmalgabe mit einer reduzierten Dosis sowohl an Cox-Inhibitoren als auch an Gestagen auskommt und gleichzeitig eine verbesserte Effizienz bei der Empfängnisverhütung im Vergleich zu den derzeit verfügbaren Gestagen basierten Präparaten für die Notfallverhütung zeigt. Eine weitere Aufgabe der Erfindung ist es Päpatate bereitzustellen, die sichere Einmalanwendung mit reduzierten Nebenwirkungen erlaubt und bei der darüber hinaus COX-Inhibitoren angewendet werden, die ein minmales oder bei Einmalgabe abwesendes Risikopotential in Bezug auf kardiale bzw. renale Effekte haben.

Diese Aufgabe wird durch die vorliegende Erfindung gelöst. Es wurde gefunden, dass COX-Inhibitoren den Effekt von Gestagenen verstärken, wodurch die zur Vermeidung der Ovulation erforderliche Dosis an Gestagen überraschenderweise deutlich reduziert werden kann. Umgekehrt können Gestagene die ovulationsinhibitorischen Effekte von COX-Inhibitoren noch verstärken. Durch diese synergistischen Wirkungen ist es möglich, trotz einer niedrigeren Dosierung, insbesondere des Gestagens, eine vergleichbare oder sogar erhöhte kontrazeptive Sicherheit zu erreichen und gleichzeitig eine Verringerung der Nebenwirkungen beider Substanzklassen zu erzielen.

Insbesondere Gestagen-assoziierte Nebenwirkungen (wie z.B. Übelkeit, Kopfschmerzen) können durch Zugabe des COX-Inhibitors reduziert werden, was die Verträglichkeit und Akzeptanz der Methode erhöht.

Wie gezeigt werden konnte, bewirkt die Verwendung von Cox-Inhibitoren auch eine Verminderung der Fertilisierungsrate von ovulierten Kumulus-Eizell Komplexen (Eizellen, die von Kumulus Zellen umgeben sind, wobei den die Eizelle umgebenen Kumulus Zellen wichtige Funktionen bei der Fertilisierung zukommen Tamba S. et al. PNAS 2008). Das heist selbst wenn die Ovulation durch Cox-Inhibitoren nicht unterdrückt wird gibt es einen weiteren kontrazeptiven Effekt durch eine verminderte Fertilisierungsrate. Verantwortlich hierfür sind vermutlich Einflüsse auf die Eigenschaften des Kumulus Expansion, die nach dem LH-Peak eintritt und bei der Prostaglandine ein Rolle spielen (siehe Beispiel 3). Dies führt zu einer verbesserten kontrazeptive Sicherheit gegenüber den LNG basierten Marktprodukten, die lediglich den Mechanismus der Ovulationshemmung nutzen. Somit wird eine verbesserte Effizienz bei der Empfängnisverhütung trotz Ovulation erreicht. Zur Wirkung der Prostaglandine auf die Fertilität sei auf den Artikel von Normann R.J. THE LANCET 2001 und Sirois J. et al. Human Reproduction Update, 2004 hingewiesen.

Grundsätzlich ist es auch möglich die Wirkstoffdosis (Gestagen und Cox-Inhibitor) zu gleichen Teilen auf 2 Pillen zu verteilen, ohne das es zu einer signifikanten Beeinträchtigung der Wirksamkeit kommt, solange sichergestellt ist, dass der Abstand zwischen der ersten und der 2-ten Dosis 12 Stunden nicht überschreitet und weiterhin gewährleistet ist, dass beide Pillen in einem Zeitraum von 72 Stunden nach dem ungeschützten Koitus eingenommen werden. Aus den in Absatz 24 genannten Gründen (Compliance bei der Einnahme der 2-ten Dosis und Patientenkomfort) ist diese Darreichungsform jedoch nicht bevorzugt.

Als Gestagen kommen grundsätzlich die bei oralen Kontrazeptiva eingesetzten bekannten sowie andere zur oralen Anwendung geeignete Gestagene in Frage, wie z.B. Chlormadinone acetat (CMA),_Norgestimate (NGM), Norelgestromin (NGMN), Norethisteron (NET) / Norethisterone-Acetat (NETA), Etonogestrel (3-keto-desogestrel), Nomegestrol-Acetat (NOMAc), Demegestone, Promegestone, Drospirenon (DRSP), Medroxyprogesteron-Acetat (MPA), Cyproteronacetat (CPA), Trimegestone (TMG), Levonorgestrel (LNG), Norgestrel (NG), Desogestrel (DSG), Gestoden (GSD) oder Dienogest (DNG), wobei, Levonorgestrel (LNG), Desogestrel (DSG), Gestoden (GSD) und Dienogest (DNG) bevorzugt sind. Für die erfindungsgemäße Verwendung als Präparat für die Notfallverhütung besonders bevorzugt ist darunter das Levonorgestrel.

Als untere Dosisgrenze für das für die Notfallverhütung eingesetzte Gestagen kommt eine Dosis zur Anwendung die gerade noch ovulationshemmend ist. Diese Dosis variiert in Abhängigkeit vom angewendeten Gestagen. In der Literatur findet man hier die folgenden orientierenden minimal Dosen für eine Ovulationshemmung (bei 21-tägiger Anwendung) (Tabelle 1):

**Tabelle 1**

| **Gestagen** | **p.o. Dosis/Tag** |
|---|---|
| Chlormadinonacetat (CMA) | 1,5-2 mg³³, 5 mg³⁰ |
| Cyproteronacetat (CPA) | 1 mg³³ |
| Desogestrel (DSG) | 60 µg²⁸ |
| Dienogest (DNG) | 1-2 mg²⁹ |
| Drospirenon (DRSP) | 2 mg³³ |
| Dydrogesteron | >30 mg³³ |
| Ethynodiol | 2 mg³³ |
| Gestoden (GSD) | 30 µg³³, 40-50 µg²⁸ |
| Levonorgestrel (LNG) | 50 µg³³, 60 µg²⁸ |
| Lynestrenol | 2 mg³³ |
| Medrogeston | 10 mg³³ |
| Medroxyprogesteronacetat (MPA) | 10 mg³³ |
| Nomegestrolacetat (NOMAc) | 2,5 mg³² |
| Norethisteron | 0,5 mg³³ |
| Norethisteronacetat (NETA) | 0,5 mg³³ |
| Norgestimat (NGM) | 180-250 µg³¹ |
| Progesteron | 300 mg³³ |
| Promegeston | 0,5 mg³³ |
| Trimegeston | 0,5 mg³³ |

Die obere Dosisgrenze beträgt bei Einmalgabe für Levonorgestrel 900 µg. Erfindungsgemäß bevorzugt ist eine Dosis von 750 µg, d.h. einer Menge, die der Hälfte der Dosierung entspricht, wie sie in heutigen Marktprodukten zur Notfallverhütung, die im übrigen alle LNG basierend sind, eingesetzt wird.

Für die weiteren erfindungsgemäß verwendeten Gestagene ergeben sich als Dosisbereiche bei vorzugsweiser Einmalgabe die folgenden Mengen:

**Tabelle 2**

| **Gestagen** | **Dosis p.o. (mg)** | |
|---|---|---|
| | Minimum | Maximum |
| Chlormadinonacetat (CMA) | 1,5 | 75 |
| Cyproteronacetat (CPA) | 1 | 15 |
| Desogestrel (DSG) | 0,06 | 0,9 |
| Dienogest (DNG) | 1 | 30 |
| Drospirenon (DRSP) | 2 | 30 |
| Dydrogesteron | 30 | 450 |
| Ethynodiol | 2 | 30 |
| Gestoden (GSD) | 0,03 | 0,75 |
| Levonorgestrel (LNG) | 0,05 | 0,9 |
| Lynestrenol | 2 | 30 |
| Medrogeston | 10 | 150 |
| Medroxyprogesteronacetat (MPA) | 10 | 150 |
| Nomegestrolacetat (NOMAc) | 2,5 | 37 |
| Norethisteron | 0,5 | 8 |
| Norethisteronacetat (NETA) | 0,5 | 8 |
| Norgestimat (NGM) | 0,18 | 3,7 |
| Progesteron | 300 | 4500 |
| Promegeston | 0,5 | 8 |
| Trimegeston | 0,5 | 8 |

Erfindungsgemäß besonders bevorzugt sind Dosisbereiche, die der doppelten bis 6-fachen Menge einer noch ovulationshemmenden Dosis entsprechen. Erfindungsgemäß besonders bevorzugte Dosisbereiche sind somit für das Levonorgestrel 100 - 360 µg, für das Dienogest 1 - 12 mg, für das Desogestrel 120 - 360 µg, für das Gestoden 60 - 300 µg, 3 - 30 mg für das CMA, 360 - 1500 µg für das NGM und 5 - 15 mg für das NOMAc.

Besonders bevorzugt ist eine Zusammensetzung zur Notfallverhütung die neben dem Cox-Inhibitor Levonorgestrel in einer Menge von 150 - 300 µg enthält.

Als Cox-Inhibitoren, die erfindungsgemäß verwendet werden, kommen grundsätzlich alle für andere Indikationen verfügbaren Cox-Inhibitoren, wie z.B. auch das Meloxicam in Frage. Erfindungsgemäß besonders geeignet sind darunter das Piroxicam, Naproxen, Celecoxib, Diclofenac, Tenoxicam, Nimesulide, Lornoxicam und Indomethacin, wobei das Piroxicam besonders bevorzugt ist. So zeigt dieser Cox Inhibitor in Kombination mit einem Gestagen (LNG) eine bessere Wirkung als sie z.B. für die Kombination von LNG mit Meloxicam (siehe Beispiel 1) erreicht wird und vermindert darüber hinaus die Fertilisierung.

Auch für die Cox-Inhibitoren sind unterschiedliche Dosierungen, in Abhängigkeit vom verwendeten Cox-Inhibitor, einzusetzen. Dosisbereiche für die erfindungsgemäß verwendeten Cox-Inhibitoren ergeben sich aus den empfohlen Höchstdosen pro Tag für den jeweiligen Cox-Inhibitor bei vorzugsweiser Einmalgabe. Diese empfohlen Höchstdosen beziehen sich auf längerfristige Behandlungen und Dauertherapien, so das für die hier vorliegende Indikation der Notvallverhütung mit bevorzugter Einmalgabe auch die dreifache Menge der empfohlenen Tageshöchsdosis Verwendung finden kann. Als Untergrenze wird ein Viertel der empfohlenen Tages Höchstdosen angesehen. Für die erfindungsgemäß verwendeten Cox-Inhibitoren ergeben sich die folgenden Mengen (Tabelle 3):

**Tabelle 3**

| | Empfohlene Tageshöchstdosis* (mg) | Bereich für die Notfallverhütung (mg) |
|---|---|---|
| Piroxicam | 20 | 5 - 60 |
| Tenoxicam | 40 | 10 - 120 |
| Naproxen | 1250 | 300 - 3800 |
| Diclofenac | 150 | 40 - 450 |
| Indomethacin | 200 | 50 - 600 |
| Celecoxib | 400 | 100 - 1200 |
| Nimesulide | 200 | 50 - 600 |
| Lornoxicam | 16 | 4 - 48 |
| Ibuprofen | 2400** | 600 - 3600 |

| | | |
|---|---|---|
| * Tageshöchstdosen gemäß Wolters Kluver Health (Ovid SP Verlag) Drug Information Full Text ** Tageshöchstdosis gemäß Packungsbeilage | | |

In einer bevorzugten Ausführungsform der Erfindung, wird in der pharmazeutischen Formulierung eine Kombination aus Piroxicam in einem Dosisbereich von 5-60 mg und Levonorgestrel im Bereich von 60-750 µg verwendet. Besonders bevorzugt ist eine Formulierung enthaltend 10-30 mg Piroxicam und 150 bis 300 µg LNG.

Die pharmazeutische Formulierung, kann in festem oder flüssigem Zustand vorliegen, zum Beispiel als Tablette, Filmtablette oder beschichtete Tablette, Wafer, Kapsel, Pille oder Pulverzubereitungen. Die lyophilisierten Pulverampullenzubereitungen - welche die in situ Herstellung von flüssigen Zusammensetzungen ermöglicht - gehören ebenfalls dazu. Flüssige Zusammensetzungen können zum Beispiel Injektions- oder Infusionslösungen sein.

Die Herstellung der pharmazeutischen Formulierung ist einem Experten geläufig. Die Herstellung einer Formulierung als Tablette ist in Beispiel 4 beschrieben.

Die Wirksamkeit der erfindungsgemäßen Zusammensetzung wurde durch ovulationsinhibitorische Studien an erwachsenen, normal zyklisierenden weiblichen Ratten festgestellt. So wurde zunächst mit 5 Dosisgruppen plus Placebo (0,003 mg, 0,01 mg, 0,03 mg, 0,1 mg und 0,3 mg), die Grenzdosis an Levonorgestrel (LNG) ermittelt, bei der noch keine ovulationsinhibitorische Wirkung auftritt. Es zeigte sich, dass eine Dosis von 0,01 mg/Tier (subkutan nachfolgend abgekürzt mit s.c.) Levonorgestrel gerade noch keine ovulationsinhibitorische Wirkung hat.

Mit der so ermittelten Dosis an LNG (0,01 mg) wurde anschließend ein Vergleichsversuch durchgeführt, bei dem die ovulationshemmende Wirkung von LNG, Placebo, Meloxicam, Piroxicam, sowie die Kombination aus LNG und Meloxicam gegen die erfindungsgemäße Zusammensetzung LNG plus Piroxicam verglichen wurde.

Es wurden mit Beginn des Metoestrus jeweils 5 Tiere an vier aufeinander folgenden Tagen mit jeweils (a) Placebo, (b) 0,01 mg LNG, (c) 2 mg Piroxicam, (d) 0,01 mg LNG und 2 mg Piroxicam (e) 2 mg Meloxicam und (f) 0,1 mg LNG und 3 mg Meloxicam behandelt. LNG wurde hierbei s.c. und der COX-Inhibitor per oral (p.o.) verabreicht.

Während in den Gruppen bei der Kontrollgruppe (a) 42 Eizellen bzw. 35 Eizellen in der Behandlungsgruppe (b) mit 0,1mg Levonorgestrel ovulierten, lag die Ovulationsrate bei einer Kombination aus 2 mg p.o. verabreichtem Piroxicam plus 0,1mg Levonorgestrel (Behandlungsgruppe d) bei nur 3 Eizellen (nur ein Tier von 5 zeigte überhaupt eine Ovulation). Bei 2 mg Piroxicam alleine (Behandlungsgruppe c) ovulierten hingegen 9 Eizellen.

Bei einer noch niedrigeren Dosis von nur 1 mg Piroxicam gewählt lag die Ovulationsrate bei der Kombination mit 0,1 mg Levonorgestrel bei 16 Eizellen, während 1 mg Piroxicam 28 Eizellen ovulieren lies.

Der Vergleich von LNG/Meloxicam gegen LNG/Piroxicam gegen LNG zeigt, dass Meloxicam plus LNG in diesem direkten Vergleich weniger effektiv ist, als Piroxicam plus LNG. So zeigte eine Kombination von 2 mg Meloxicam plus 0,01 mg Levonorgestrel noch immer ein Ovulationsrate von 18 Eizellen bei 5 Tieren.

In einem weiteren Versuch (Beispiel 2) mit intakten zyklischen Ratten (n=10 Tieren) konnte überraschend gezeigt werden, das Piroxicam alleine einen signifikanten dosisabhängigen Effekt auf die Serum Progesteron Konzentration am Abend (19.00 Uhr) des Pro-Östrus, also zum Zeitpunkt des LH-Peaks, hat. Dies kann als Indikator für die Effizienz des LH-Peaks zur Umstellung von einem vornehmlich Östrogen produzierenden Ovar (vor dem LH-Peak) auf ein vornehmlich Progesteron produzierendes Ovar (den LH-peak bedingt) gesehen werden. Dieser überraschende Befund, zeigt das Piroxicam alleine einen Effekt auf die hormonelle Situation hat, der einen kontrazeptiven Effekt oder eine Verstärkung eines kontrazeptiven Effekts durch ein Gestagen bewirken kann. In Gegensatz hierzu ist in der Literatur beschrieben, das andere Cox-Inhibitoren keinen Einfluss auf die hormonellen Spiegel haben [Pall et al.; Human Reproduction Vol 16, Nr. 7, pp 1323-1328 (2001); Bata et al., J Clin Pharmacol (2006) 46:925-932]. Auch in diesem Versuch zeigte sich, das die Kombination von niedrigdosiertem Levonorgestrel (0,01 mg s.c.) mit Piroxicam (0,5; 1 und 2 mg p.o.) die Ovulation deutlich besser hemmt als jede Substanz für sich alleine: In der Vehikel Gruppe ovulierten 100 Eizellen, mit LNG alleine 68, bei 0,5mg Piroxicam 85, bei 1 mg Piroxicam 56 und bei 2mg Piroxicam 30. Die Ovulationsraten in der Kombination mit 0,01 mg Levonorgestrel und 0,5mg Piroxicam (31 Eizellen), mit 1 mg Piroxicam (19 Eizellen und mit 2mg Piroxicam (0 Eizellen) lagen hier deutlich geringer, bzw. führten in der höchsten Dosis sogar zur völligen
Anovulation (jeweils 10 Tiere pro Gruppe).

Die Erfindung wird durch die nachfolgenden, nicht limitierenden Beispiele erläutert.

### Beispiel 1 (Ovulationshemmtest bei der Ratte):

Zum Nachweis ovulationshemmender Substanzen ist die Ratte ein besonders geeignetes Tiermodel, da sie spontan ovuliert und der Zyklus sich anhand von Vaginalabstrichen leicht beobachten lässt.

Im nachfolgenden Versuch wurden weibliche Ratten mit einem Gewicht von 200-220 g eingesetzt. Die Tiere wurden in Makrolonkäfigen in kontrolliert belichteten Räumen (12 Std. Dunkelheit: 12 Std. Helligkeit) gehalten, mit einer Standard-Diät ernährt und mit Wasser ad libitum getränkt.

Levonorgestrel wurden in Benzylbenzoat/Rizinusöl (1+4 v/v) gelöst und die tägliche Dosis in einem Volumen von 1 ml /kg Körpergewicht s.c. appliziert.

Die COX-Inhibitoren wurden in einer Trägerflüssigkeit (85 mg MyrjR53 (2-hydroxyethyl octadecanoate; CAS Nr. 9004-99-3) in 100 ml 0,9 % w/v NaCl-Lösung) suspendiert und die der Behandlungsgruppe entsprechende Tagesdosis in einem Volumen von 2 ml / kg Körpergewicht oral verabreicht.

Es wurden zwei Zyklen anhand von Vaginalabstrichen vor Versuchsbeginn beobachtet. Nur Tiere mit einem regelmäßigen 4-Tage-Zyklus kommen in den Versuch. Die Zuordnung zu den Behandlungsgruppen erfolgt randomisiert. Im Metoestrus beginnend, wird die Testsubstanz über 4 Tage (Tag 1-4) appliziert und weiterhin der Zyklus kontrolliert. An Tag 3 der Behandlung (Proöstrus) erfolgt um 9.00 Uhr und ab 18.30 Uhr eine retrobulbäre Blutentnahme zur Bestimmung des Luteinisierenden Hormons (LH). An Tag 4 (nach der Applikation) werden die Tiere, die beim Vaginalabstrich einen Östrus oder Metöstrus haben, einseitig unter Narkose ovarektomiert. Von den Tuben werden Quetschpräparate angefertigt und mikroskopisch auf das Vorhandensein von Eizellen untersucht. Am Tag 5 werden alle Tiere (intakte und halbseitig ovarektomierte) getötet, die Tuben der östrischen und metöstrischen Tiere werden präpariert und in gleicher Weise untersucht.

Die durchgeführten Versuche zeigen, dass eine niedrige Dosis von Levonorgestrel, die selbst noch nicht ovulationsinhibitorisch wirkt, in Kombination mit Cox-Inhibitoren eine verstärkte Ovulationsunterdrückung bewirkt, wobei im Falle von Piroxicam eine nahezu vollständige Ovulationsunterdrückung erreicht werden kann. Die Cox-Inhibitoren hingegen bewirken alleine nur eine partielle Ovulationsunterdrückung.

Die Ergebnisse aus der Studie, d.h. die Kombinationswirkung von Levonorgestrel plus Cox-Inhibitoren auf die Ovulation, sind in Tabelle 4 zusammengefasst:

**Tabelle 4**

| Behandlungsgruppe | Ovulationszahlen absolut | Ovulationszahl Mittelwert |
|---|---|---|
| Vehikel | 42 | 8,4 ± 0,5 |
| LNG 0,01 mg s.c. | 35 | 7,0 ± 2,0 |
| Meloxicam 2mg p.o. | 28 | 5,6 ± 2,5 |
| Piroxicam 2mg p.o. | 9 | 1,8 ± 0,4 |
| LNG (0,01 mg)+ Priox (2mg) | 3 | 0,6 ± 1,3 (nur 1 Tier ovulierte 3 Eizellen) |
| LNG (0,01 mg) + Melox (2mg) | 18 | 3,6 ± 3,3 |

### Beispiel 2

Der Versuchsaufbau entspricht dem in Beispiel 1 beschriebenen.

Die Ergebnisse zu den Progesteronkonzentrationen im Pro-Östrus um 19.00 Uhr sind in Tabelle 5 zusammengefaßt:

**Tabelle 5**

| Behandlungsgruppe | Progesterone im Pro-Östrus um 19.00 Mittelwert ± SD (n=10) |
|---|---|
| Vehikel | 254 ± 88 |
| Piroxicam 0,5mg p.o. | 237 ± 49 |
| Piroxicam 1 mg p.o. | 183 ± 67 |
| Piroxicam 2mg p.o. | 122 ± 94 |

### Beispiel 3

Substanzen können dadurch Einfluss auf die Fertilität nehmen, das sie die Fertilisierbarkeit von Eizellen oder Kumulus-Eizellkomplexen herabsetzen. Um solche Wirkungen zu untersuchen, können Substanzen in vivo verabreicht werden und nach erfolgter Ovulation von Kumulus-Eizellkomplexen einer in vitro Fertiliserung unterzogen werden. Die in vitro Fertilisierungsrate, wobei keine Testsubstanz mehr zugegen wird, erlaubt Rückschlüsse auf die in vivo Effekte der Testsubstanzen.

Immature weiblichen Mäusen (Stamm: B6D2F1, Charles River, Suelzfeld, Alter: 19-25 Tage) wurden in Makrolonkäfigen in kontrolliert belichteten Räumen (12 Std. Dunkelheit: 12 Std. Helligkeit) gehalten, mit einer Standard-Diät ernährt und mit Wasser ad libitum getränkt.

Die Mäuse wurden mit PMSG (Pregnant Mare Serum Gonadotropin) geprimt (10 IU/Tier i.p.). Nach 48 Stunden wurde bei den Tieren durch eine Gabe von 10 IU/Tier i.p. ein die Ovulation auslösender Stimulus erzeugt. Die COX-Inhibitoren wurden in einer Trägerflüssigkeit (85 mg MyrjR53 (2 hydroxyethyl octadecanoate; CAS Nr. 9004-99-3) in 100 ml 0,9 % w/v NaCl-Lösung) suspendiert und die der Behandlungsgruppe (n=5 Tiere pro Gruppe) entsprechende Tagesdosis in einem Volumen von 0,2ml p.o. 8 Stunden vor und zusammen mit hCG appliziert. Vierzehn Stunden nach hCG-Gabe wurden die Tiere getötet. Ovulierte Eizellen und Kumulus-Eizellkomplexe wurden aus der Bursa Ovarii und/oder Ovidukt gewonnen und einer in vitro Fertilisierung unterzogen, wobei für die Fertilisierung eine Spermienzahl von 40000 Spermien/0,5 ml für 1 Stunde eingesetzt wurde. Vierundzwanzig Stunden nach der Inkubation mit den Spermien wird die Anzahl der fertilisierten Eizellen festgestellt und die Prozentduale Fertilisierungsrate bestimmt.

Die Ergebnisse zeigen, das Piroxicam einen Einfluss auf die Fertilisierbarkeit ovulierter Kumulus-Eizell Komplexe hat.

Die Ergebnisse aus der Untersuchung des Einflusses von Piroxicam auf die Fertilisierungsrate von ovulierten Kumulus-Eizell Komplexen sind in Tabelle 6 zusammengefasst:

**Tabelle 6**

| Behandlungsgruppe | Fertilisierungsrate (% ± SD) |
|---|---|
| Vehikel | 55 ± 16 |
| Piroxicam (2x0,5 mg/Tier p.o.) | 12 ± 7 |
| Piroxicam (2x0,3 mg/Tier p.o.) | 14 ± 18 |
| Piroxicam (2x0,15 mg/Tier p.o.) | 19 ± 8 |

### Beispiel 4 (Verfahren zur Herstellung einer Tablette für die Notfallverhütung)

Tabletten mit einer Gesamtmasse von 200 mg pro Tablette und der in Tabelle 7 angegebenen Zusammensetzung

**Tabelle 7**

| | |
|---|---|
| Levonorgestrel, mikronisiert | 0,25 mg |
| Piroxicam, mikronisiert | 20,00 mg |
| Lactose Monohydrat | 107,75 mg |
| Maisstärke | 36,00 mg |
| Modifizierte Stärke | 24,00 mg |
| Polyvinylpyrrolidon 25,000 | 10,00 mg |
| Magnesiumstearat | 2,00 mg |

wurden hergestellt, durch Befüllung eines Wirbelschichtgranulators mit 31,68 kg Maisstärke, 21,12 kg modifizierter Stärke, 0,22 kg Levonorgestrel, mikronisiert, 17,6 kg Piroxicam, mikronisiert and 94,82 kg Lactose Monohydrat und Aktivierung der Wirbelschicht. Eine wässrige Lösung von 8,8 kg Polyvinylpyrrolidon 25,000 in 50 kg gereinigtem Wasser wurde kontinuierlich auf die Wirbelschicht gesprüht, bei gleichzeitiger Trocknung der Mischung durch Erhitzen des Luftstroms der Wirbelschicht. Am Ende des Prozesses werden 1,76 kg Magnesiumstearat in den Wirbelschichtgranulator gebracht und mit den entstandenen Granulen bei laufender Wirbelschicht gemischt. Das so gebildete Granulat wurde in einer Rundläufertablettenpresse inTablettenform mit 8 mm Durchmesser gepresst.

### Literaturverzeichnis

1. Swahn ML. Effect of post-coital contraceptive methods on the endometrium and the menstrual cycle. Acta Obstet Gynecol Scand 1996;75:738-44.
2. Ling WY,. Mode of action of dl-norgestrel and ethinylestradiol combination in postcoital contraception. Fertil Steril 1979;32:297-302.
3. Rowlands S,. A possible mechanism of action of danazol and an ethinylestradiol/norgestrel combination used as postcoital contraceptive agents. Contraception 1986;33:539-45.
4. Croxatto HB,. Effects of the Yuzpe regimen, given during the follicular phase, on ovarian function. Contraception 2002;65:121-8.
5. Kubba AA,. The biochemistry of human endometrium after two regimens of postcoital contraception: a dl-norgestrel/ethinylestradiol combination or danazol. Fertil Steril 1986:45:512-6.
6. Ling WY. Mode of action of dl-norgestrel and ethinylestradiol combination in postcoital contraception. II. Effect of postovulatory administration on ovarian function and endometrium. Fertil Steril 1983;39:292-7.
7. Yuzpe AA,. Post coital contraception-a pilot study. J Reprod Med 1974; 13:53-8.
8. Taskin O. High doses of oral contraceptives do not alter endometrial α1 and αvβ3integrins in the late implantation window. Fertil Steril 1994,61:850-5.
9. Raymond EG. Effect of the Yuzpe regimen of emergency contraception on markers of endometrial receptivity. Hum Reprod 2000;15:2351-5.
10. Ling WY. Mode of action of dl-norgestrel and ethinylestradiol combination in postcoital contraception. III. Effect of preovulatory administration following the luteinizing hormone surge on ovarian steroidogenesis. Fertil Steril 1983;40:631-6.
11. Croxatto HB. Mechanism of action of hormonal preparations used for emergency contraception: a review of the literature. Contraception 2001 ;63:111-21.
12. Croxatto HB. Mechanisms of action of emergency contraception. Steroids 2003;68:1095-8. 5
13. Glasier A. Emergency postcoital contraception. N Engl J Med 1997; 337:1058-64.
14. Trussell J, Raymond EG. Statistical evidence concerning the mechanism of action of the Yuzpe regimen of emergency contraception. Obstet Gynecol 1999;93:872-6.
15. Hapangama D. The effects of peri-ovulatory administration of levonorgestrel on the menstrual cycle. Contraception 2001 ;63:123-9.
16. Durand M. On the mechanisms of action of short-term levonorgestrel administration in emergency contraception. Contraception 2001;64:227-34.
17. Marions L. Emergency contraception with mifepristone and levonorgestrel: mechanism of action. Obstet Gynecol 2002;100:65-71.
18. Marions L. Cekan SZ, Bygdeman M, Gemzell-Danielsson K. Effect of emergency contraception with levonorgestrel or mifepristone on ovarian function. Contraception 2004;69:373-7.
19. Croxatto HB. Pituitary-ovarian function following the standard levonorgestrel emergency contraceptive dose or a single 0.75-mg dose given on the days preceding ovulation. Contraception 2004;70:442-50.
20. Okewole IA. Effect of single administration of levonorgestrel on the menstrual cycle. Contraception 2007;75:372-7.
21. Durand M. Late follicular phase administration of levonorgestrel as an emergency contraceptive changes the secretory pattern of glycodelin in serum and endometrium during the luteal phase of the menstrual cycle. Contraception 2005;71:451-7.
22. Nascimento JA. In vivo assessment of the human sperm acrosome reaction and the expression of glycodelin-A in human endometrium after levonorgestrelemergency contraceptive pill administration. Hum Reprod 2007;22:2190-5.
23. Kesserü E. The hormonal and peripheral effects of dnorgestrel in postcoital contraception. Contraception 1974;10:411-24.
24. Trussell J. Plan B and the politics of doubt. J Am Med Assoc 2006;296:1775-8.
25. Müller AL,. Postcoital treatment with levonorgestrel does not disrupt postfertilization events in the rat. Contraception 2003;67:415-19.
26. Ortiz ME. Postcoital administration of levonorgestrel does not interfere with post-fertilization events in the new-world monkey Cebus apella. Hum Reprod 2004;19:1352-6.
27. Hatcher RA, Trussell J,. Emergency Contraception: The Nation's Best Kept Secret. Decatur GA: Bridging the Gap Communications, 1995.
28. Teichmann gibt in "Empfängnishütung: eine vergleichende Übersicht aller Methoden, Risiken und Indikationen", erschienen im Georg Thieme Verlag (1996)
29. Moore, C, Carol, W, Gräser, T, et al. in Clin Drug Invest 18, 271-8 (1999) Influence of dienogest on ovulation in young fertile women
30. R. Druckmann; contraception 79 (2009) 272 - 281: Profile of progesterone derivative chlormadinone acetate - Pharmacodynamic properties and therapeutic applications
31. Fertility control Stephen L. Corson, Richard J. Derman, Louise B. Tyrer Edition: 2, illustrated Veröffentlicht von Taylor & Francis, 1994 ISBN 096979780X, 9780969797807 514 Seiten
32. Bazin B, Thevenot R, Bursaux C and Paris J (1987) Effect of nomegestrol acetate, a new 19 nor-progesterone derivative, on pituitary-ovarian function in women. Br J Obstet Gynaecol 94,1199-1204
33. Schindler A.E. et al (2003) Classification and pharmacology of progestins Maturitas 46 S1, 7-16

### Gegenstände:

Gegenstand 1: Pharmazeutische Zusammensetzung als Einzelverabreichungsdosis, **dadurch gekennzeichnet,** dass sie
(a) 50-900 µg Levonorgestrel, 1-30 mg Dienogest, 60-900 µg Desogestrel, 30-750 µg Gestoden, 1,5-75 mg CMA, 180-3700 µg NGM, 1-15 mg Cyproteronacetat (CPA), 2-30 mg Drospirenon (DRSP), 30-450 mg Dydrogesteron, 2-30 mg Ethynodiol, 2-30 mg Lynestrenol, 10-150 mg Medrogeston, 10-150 mg Medroxyprogesteronacetat (MPA), 0,5-8 mg Norethisteron, 0,5-8 mg Norethisteronacetat (NETA), 300-4500 mg Progesteron, 0,5-8 mg Promegeston, 0,5-8 mg Trimegeston oder 2,5-37 mg NOMAc und
(b) als COX-Inhibitor Piroxicam , Naproxen, Celecoxib, Indomethacin, Diclofenac, Nimesulide, Lornoxicam oder Tenoxicam als Wirkstoffe und die bekannten in der pharmazeutischen Praxis verwendeten Hilfsstoffe, enthält.
Gegenstand 2: Pharmazeutische Zusammensetzung nach Gegenstand 1, **dadurch gekennzeichnet,** dass sie als Gestagen 100 - 360 µg Levonorgestrel, 2 - 12 mg Dienogest, 120 - 360 µg Desogestrel, 60 - 300 µg Gestoden, 3 - 30 mg CMA, 360 - 1500 µg NGM oder 5 - 15 mg NOMAc enthält.
Gegenstand 3: Pharmazeutische Zusammensetzung nach Gegestand 1 oder 2, **dadurch gekennzeichnet,** dass der COX-Inhibitor folgenden Mengebereichen enthalten ist:

| | |
|---|---|
| Piroxicam | 5-60 mg |
| Tenoxicam | 10-120 mg |
| Naproxen | 300-3800 mg |
| Diclofenac | 40-450 mg |
| Indomethacin | 50-600 mg |
| Celecoxib | 100-1200 mg |
| Nimesulide | 50 - 600 mg |
| Lornoxicam | 4 - 48 mg |

Gegenstand 4: Pharmazeutische Zusammensetzung nach Gegenstand 1 - 3 enthaltend als Gestagen Levonorgestrel.
Gegenstand 5: Pharmazeutische Zusammensetzung nach Gegenstand 1 - 4, enthaltend Levonorgestrel in einer Dosis von 150-300 µg.
Gegenstand 6: Pharmazeutische Zusammensetzung nach Gegenstand 1 - 4 enthaltend als COX-Inhibitor Piroxicam.
Gegenstand 7: Pharmazeutische Zusammensetzung nach Gegenstand 5 enthaltend Piroxicam in einer Dosis von 5-60 mg.
Gegenstand 8: Pharmazeutische Zusammensetzung nach Gegenstand 5 enthaltend Piroxicam in einer Dosis von 10-30 mg.
Gegenstand 9: Pharmazeutische Zusammensetzung nach Gegenstand 1 - 4 enthaltend als Gestagen Levonorgestrel und als Cox-Inhibitor Piroxicam.
Gegenstand 10: Pharmazeutische Zusammensetzung nach Gegenstand 9 enthaltend 150 - 300 µg Levonorgestrel und 10 - 30 mg Piroxicam.
Gegenstand 11: Verwendung von pharmazeutischen Zusammensetzungen nach einem der Gegenstände 1-8 zur Herstellung eines Arzneimittels zur Schwangerschaftsverhütung im Notfall.
Gegenstand 12: Verwendung gemäß Gegenstand 9, wobei die pharmazeutische Zusammensetzung in einer Einzelverabreichungsdosis innerhalb von bis zu 72 Stunden nach dem Koitus verabreicht wird.
Gegenstand 13: Pharmazeutische Zusammensetzung nach Gegenstand 1 oder 2, dadurch gekennzeichnet, dass als COX-Inhibitor Ibuprofen in einer Menge von 600 - 3.600 mg enthalten ist.

## Patentansprüche

1. Pharmazeutische Zusammensetzung als Einzelverabreichungsdosis,
**dadurch gekennzeichnet, dass** sie
(a) 50-900 µg Levonorgestrel, und
(b) als COX-Inhibitor, , 50-600 mg Indomethacin oder 40-450 mg Diclofenac, als Wirkstoffe und die bekannten in der pharmazeutischen Praxis verwendeten Hilfsstoffe, enthält.

2. Pharmazeutische Zusammensetzung nach Anspruch 1, **dadurch gekennzeichnet, dass** sie als Gestagen 100 - 360 µg Levonorgestrel, enthält.

3. Pharmazeutische Zusammensetzung nach Anspruch 1 oder 2, enthaltend Levonorgestrel in einer Dosis von 150-300 µg.
